# EUROPEAN PATENT SPECIFICATION

(11) **EP 3 103 401 B1**
(45) Date of publication and mention of the grant of the patent: **14.03.2018**
(21) Application number: 16173482.7
(22) Date of filing: 08.06.2016
(51) Int. Cl.: A61B 17/11, A61B 17/295, A61B 17/072, A61B 17/00, A61B 17/29

(54) **SURGICAL INSTRUMENT FOR COLON-RECTUM OPERATIONS**
CHIRURGISCHES INSTRUMENT FÜR DICKDARM-ENDDARMOPERATIONEN
INSTRUMENT CHIRURGICAL POUR DES OPÉRATIONS COLON-RECTUM

(30) Priority: 08.06.2015 IT UB20150982
(43) Date of publication of application: 14.12.2016
(73) Proprietor: Universita Degli Studi Di Perugia, 06123 Perugia (IT)
(72) Inventor: RONDELLI, Fabio, 06123 Perugia (IT)
(74) Representative: Brunacci, Marco

(56) References cited:
- EP-A1- 2 253 279
- WO-A1-01/66025
- WO-A1-2012/149643
- US-A- 5 542 949
- US-A1- 2013 197 516

## Description

This patent application for industrial invention relates to a surgical instrument for colon-rectum operation.

The malignant tumor of the colon and rectum is the fourth most common form of cancer in the world and ranks second as regards male cancer deaths and third as regards female cancer deaths.

Colon-rectum resection operations are known to remove the tumor tissue. However, despite the considerable progress made over recent years, both from a surgical point of view (standardization of Total Mesorectal Escission or TME) and from that of tumor radiotherapy (increasingly more effective neo-adjuvant and adjuvant therapies), local recurrences continue to affect a significant number of patients, particularly after performing mechanical anastomosis. Local recurrence is, firstly, clearly associated with a reduction in long-term survival and the quality of the patient's remaining life, and secondly, also a major clinical problem to be addressed (surgery or chemotherapy), with the very considerable costs for the health system. Extensive scientific evidence shows that local recurrence is linked to the system of exfoliated tumor cells within the intestinal lumen. Repeated studies, conducted both in vitro and in vivo, have shown that the washing of the lower rectal stump, where the anastomosis is performed, if conducted with cytotoxic agents and with adequate amounts of liquid (by means of the simple mechanical washing action), results in a statistically significant reduction in terms of local recurrence.

Nevertheless, currently, in daily clinical practice, such procedure of washing the rectal stump, during the resection of the colon-rectum due to cancer, performed with mini-invasive techniques, is not implemented in a systematic way. One possible explanation for this may lie in the current lack of laparoscopic forceps able to permit, with the standard positioning of the trocars at abdominal level, performing the washing operation without resorting to more extensive laparotomy incisions or in less suited locations.

Mini-invasive surgery (laparoscopic and/or robotic) has shown itself to be superior in terms of more rapid functional recovery, less surgical trauma, lower wall infections and reduced hospitalization, compared to traditional open surgery; all this, while maintaining the same oncological radicality, in terms of long-term survival and local recurrence. For this reason, in recent years, there has been a progressive increase in the number of operations using the mini-invasive technique, to the detriment of the conventional procedure. Nevertheless, currently, with laparoscopy surgery, it is possible to perform the resection of the colon-rectum to remove the tumor tissue, but it is not possible to perform washing of the colon-rectum. A recent scientific work conducted on a large sample of English colon-proctological surgeons confirmed that, despite the majority of them perform the washing procedure of the rectal stump in open surgery, because they believe it clinically beneficial for their patients to use this method, these same surgeons however overlook carrying out such action when performing mini-invasive surgery.

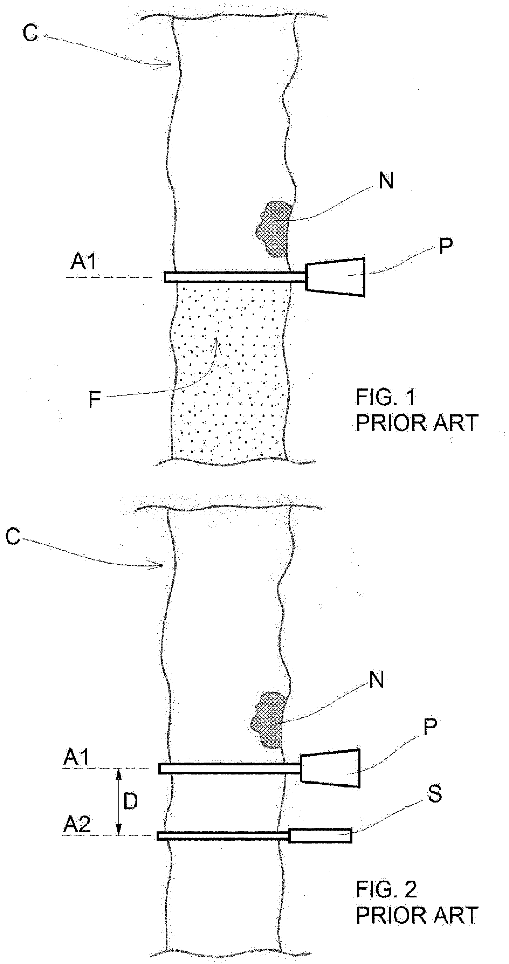

With the aid of figures 1-4 a washing and resection operation is described of the colon-rectum, in open surgery, according to the known technique.

A cut is performed in the abdomen of the patient so as to leave uncovered the colon-rectum C stump wall, in which the tumor tissue N is located.

With reference to Fig. 1, forceps (P) are used by the surgeon so as to tighten and strangulate a section of the colon-rectum stump, along a median plane A1, under the tumor tissue N. The forceps (P) are commonly called Collin type T forceps. Subsequently, the colon-rectum stump is washed, by means of a rectal enema. This way, a washing fluid (F) penetrates from the bottom to the top, in the lower part of the colon-rectum stump, below the median plane A1. The grip of the forceps (P) strangulating the colon-rectum stump along the median plane A1, prevents the washing liquid from reaching the tumor tissue N.

With reference to Fig. 2, once washing has been performed, a mechanical stapler (S), of the cut-and-sew type, is used by the surgeon to dissect and suture a section of the colon-rectum stump, along a lower plane A2, below the median plane A1 in which the colon-rectum is tightened by the forceps (P). Usually the distance D between the median plane A1 and the lower plane A2 must be between 4 and 7.5 mm, preferably 6.5 mm.

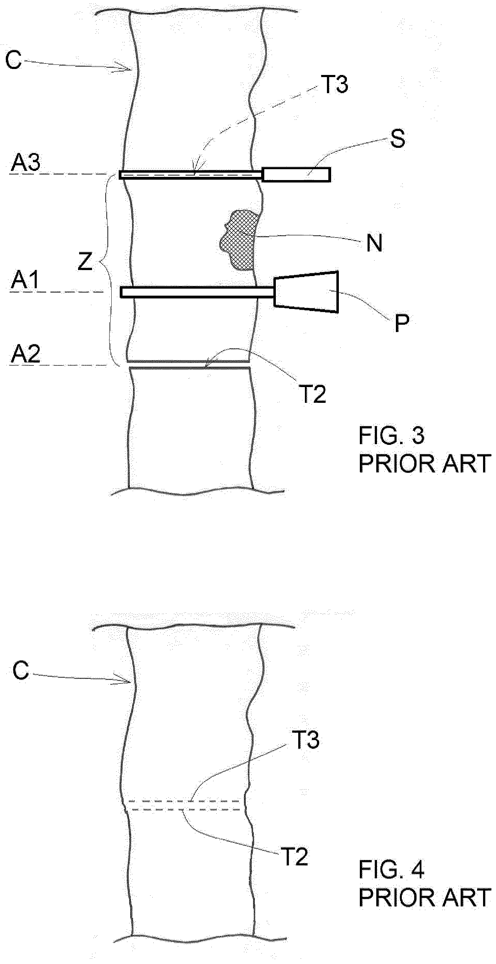

Fig. 3 shows a lower suture (T2) of the colon-rectum near the sectioned part. It should be considered that thanks to the washing of the colon-rectum, we can be sure that the lower suture (T2) takes place on a perfectly clean part of the colon-rectum stump, in an area decontaminated from exfoliated tumor cells, thus ensuring greater oncological radicality.

At this point, a mechanical stapler (S) is used by the surgeon in order to dissect and suture an upper section of the colon-rectum stump, along an upper plane (A3), above the tumor tissue N. The upper suture (T3) has been shown by means of a dotted line under the stapler (S). This way, a portion (Z) of the colon-rectum stump is removed, between the lower plane A2 and the upper plane (A3) where the tumor tissue N is located.

With reference to Fig. 4, in a way known in itself, the lower suture (T2) is joined to the upper suture (T3) so as to ensure the continuity of the colon-rectum stump.

As previously mentioned, such surgical operation cannot be performed in laparoscopy, since no forceps (P) are known for laparoscopy, i.e., adapted to be inserted in laparoscopic trocars.

Mechanical staplers (S) for laparoscopy are instead known, adapted to be inserted in trocar. Therefore if a surgical resection of the colon-rectum has to be performed in laparoscopy, the washing operation must be relinquished which, as previously said, plays a very important role to prevent local recurrence, or else additional abdominal incisions must be envisaged for the insertion of surgical instruments used in open surgery, thus effectively cancelling or greatly reducing some of the major advantages of mini-invasive surgery, previously highlighted. Another drawback of the described prior art is represented by the fact that, during the operation, the surgeon has both hands engaged: one hand to hold the forceps (P) and the other hand to hold the mechanical stapler (S).

Another drawback is represented by the fact that the washing, being performed from bottom to top is not efficient, or rather such washing, despite its efficacy demonstrated in several scientific works, may not be complete or in some way hindered by possible fecal residues.

The document EP 2253279 illustrates a surgical instrument comprising a handle, a shaft assembly that frontally protrudes from the handle, a first head that frontally protrudes from the shaft assembly and comprising a mechanical stapler adapted to cut and suture the colon-rectum; second head that frontally protrudes from the shaft assembly disposed at the side of the first head, and comprising forceps adapted to tighten and strangulate the colon-rectum without cutting it.

A drawback in using the surgical instrument described in EP 2253279 is that it is not useful for washing the colon-rectum during the surgery.

The object of the present invention is to eliminate the drawbacks of the prior art by providing a surgical instrument for colon-rectum operation which is adapted for use in mini-invasive laparoscopy surgery and at the same time allows performing both the effective washing of the rectal stump and the resection of the rectal stump section where the neoplasia is located.

Another object of the present invention is to provide such a surgical instrument for colon-rectum operation that can be used by the surgeon using just one hand and allows performing both the washing of the rectal stump and the resection of the rectal stump section where the neoplasia is located.

These objects are achieved according to the invention with the characteristics of the independent claim 1.

Convenient embodiments of the invention are evident from the dependent claims.

The surgical instrument for colon-rectum operation, according to the invention, comprises:
- a handle,
- a shaft assembly that frontally protrudes from the handle,
- a first head that frontally protrudes from the shaft assembly; said first head comprising a mechanical stapler of the "cut-and-sew" type, adapted to cut and suture the colon-rectum, and
- a second head that frontally protrudes from the shaft assembly, disposed at the side of the first head; said second head comprising forceps, adapted to tighten and strangulate the colon-rectum without cutting it.

The shaft assembly and said first head and second head are dimensioned so as to be inserted into a cannula of a trocar for laparoscopy.

Advantageously, the forceps of the second head comprise a plurality of nozzles connected to a duct formed in the second head and communicating with a duct formed in said shaft assembly.

A supply unit is connected to the duct of the shaft assembly to supply the washing fluid coming out of the nozzles to wash the colon-rectum stump from top to bottom. The nozzles of the forceps of the second head are configured so as to pierce the wall of the colon-rectum, but not cross the colon-rectum from side to side.

The diffusion of the liquid can thus only take place towards the inside of the colon lumen. In fact the closing of the branch in which the nozzles are positioned is airtight towards the outside, i.e., totally containing. All this ensures that no washing liquid or colon lumen content spreads into the abdomen.

The advantages of the surgical instrument according to the invention appear evident. In fact, to perform a colon-rectum resection operation, it is sufficient to make a small abdominal hole by means of a laparoscopy trocar. Then the two heads and the shaft assembly of the surgical instrument according to the invention are inserted into the trocar cannula, so that when the two heads reach the operation area, the stapler of the first head is below the forceps of the second head. The forceps of the second head tighten the colon-rectum stump under the tumor tissue so as to be able to perform the washing of the colon-rectum stump from top to bottom, by means of the washing liquid injected from the nozzles of the forceps. Subsequently, the stapler cuts and sutures the colon-rectum stump in the washed part, decontaminated under the forceps of the second head.

This instrument furthermore ensures a wash from top to bottom, i.e. in the direction of the normal intestinal peristalsis (oral-aboral direction), therefore this type of washing cannot but improve the elimination of the malignant cells exfoliated inside the colon lumen, compared with traditional washing which is also performed in traditional open surgery.

Additional characteristics of the invention will appear clearer from the detailed description that follows, referring to a purely exemplifying and therefore nonlimiting embodiment, illustrated in the enclosed drawings, in which:
Figs. 1 - 4 are four schematic views, illustrating washing and resection open surgery of the colon-rectum, according to the prior art;
Fig. 5 is a perspective view, illustrating the surgical instrument according to the invention, in which the forceps are open;
Fig. 5A is an enlarged detail of Fig. 5;
Fig. 6 is a view like Fig. 5, in which the forceps are closed;
Fig. 7 is a view like Fig. 5, in which the inclination has been changed of the forceps and of the stapler with respect to the shaft of the surgical instrument;
Fig. 8 is a schematic view of washing and resection surgery of the colon-rectum according to the invention; and
Fig. 9 is a sectional view, taken along the section plane IX-IX of Fig. 8.

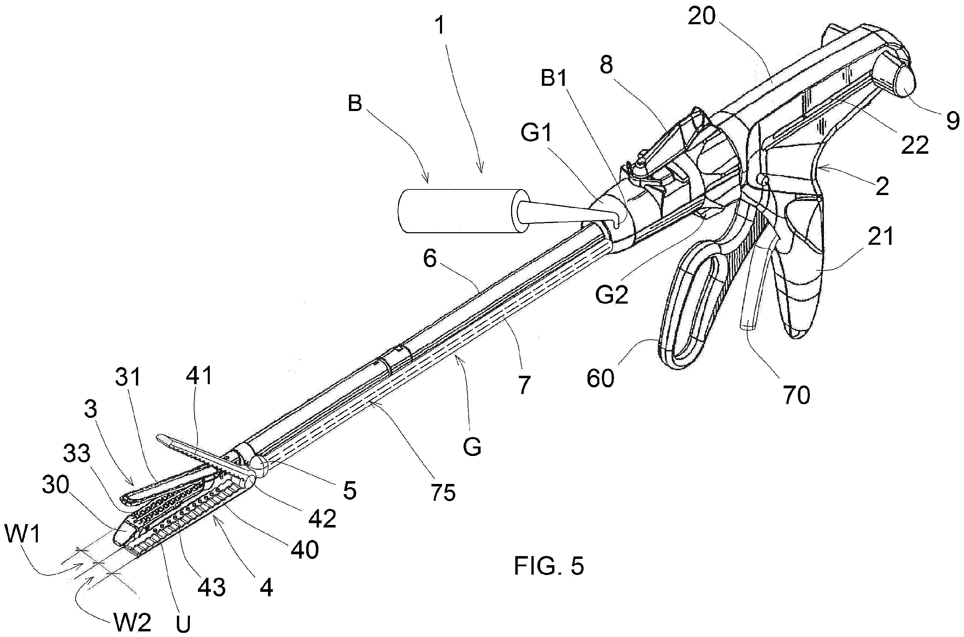

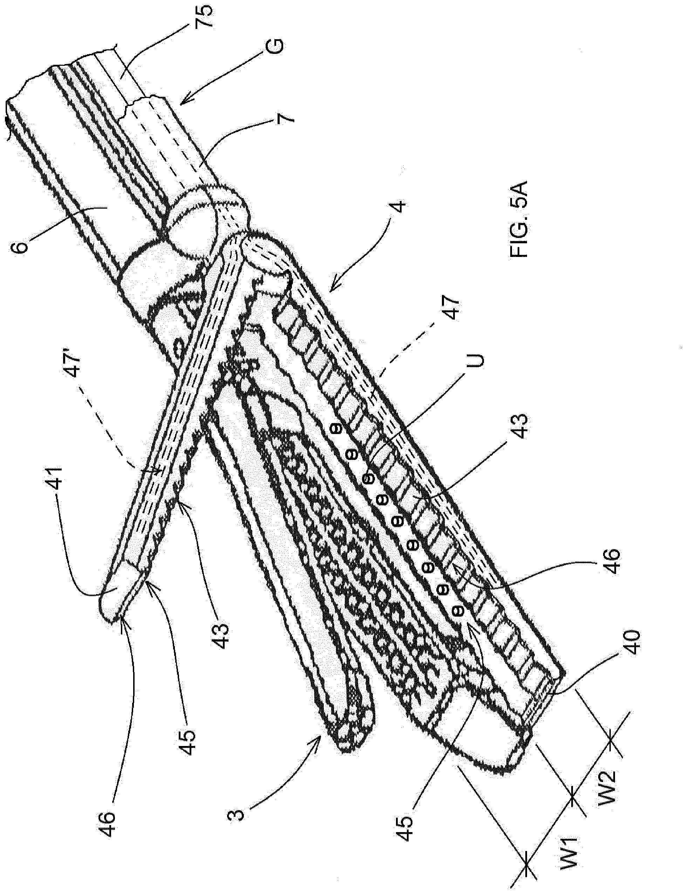

With the aid of Figures 5 to 7 the surgical instrument is described according to the invention, globally indicated by reference number 1.

The surgical instrument 1 comprises a handle 2, a shaft assembly G that frontally protrudes from the handle, a first head 3 and a second head 4 disposed side-by-side, that frontally protrudes from the shaft assembly G.

The shaft assembly G, the first head 3 and the second head 4 have such dimensions to be inserted into a trocar for laparoscopy. That is to say, the cross section of the shaft assembly and of the two side-by-side heads 3, 4 must be of such dimensions as to be inserted into a cannula of a trocar for laparoscopy having an inside diameter of less than 15 mm.

The first head 3 comprises a mechanical stapler of 'cut-and-sew' type. The stapler of the first head 3 comprises a first jaw 30 and a second jaw 31 hinged to the first jaw. The first jaw 30 of the stapler comprises a housing within which is arranged a reload 33 comprising a cutting blade to cut the tissue and a plurality of clips to suture the tissue in the proximity to the cut. For example the reload 33 can have two or three rows of clips on one part and on the other with respect to the cutting blade.

The second head 4 comprises forceps, similar to Collin type T forceps. The forceps of the second head 4 comprise a first jaw 40 and a second jaw 41 hinged to the first jaw 40, by means of a pin 42.

With reference to Fig. 5A, the two jaws of the forceps have a first longitudinal stretch 45 near the first head 3 and a second longitudinal stretch 46 parallel to the first longitudinal stretch and further away from the first head. The second longitudinal stretches 46 of the two jaws have opposite and complementary serrated surfaces 43, adapted to tighten the colon-rectum stump, so as to strangulate without cutting it. Each serrated surface 43 has a triangular wave shape in longitudinal section. The second longitudinal stretches 46 of the two jaws have opposing serrated surfaces which must ensure an airtight seal of the colon, without affecting in any way the continuity of the colon wall and its vitality, i.e., they must close, without either piercing or making ischemic.

The first longitudinal stretch 45 of the first jaw 40 comprises a plurality of nozzles U protruding upwards, adapted to pierce the tissue of the colon-rectum and to inject washing liquid inside the colon-rectum. The nozzles U are small cylindrical shaped ducts, through which the washing liquid comes out.

The nozzles U are arranged in an intermediate position of the first stretch 45 of the first jaw. The distal and proximal ends of the first stretch 45 of the first jaw are therefore without nozzles, to prevent the washing liquid from being injected out of the colon-rectum.

The first longitudinal stretch 45 of the second jaw 41 comprises an abutment surface adapted to interface with the nozzles U so as to ensure the nozzles U pierce the colon-rectum, but not cross the colon-rectum from side to side.

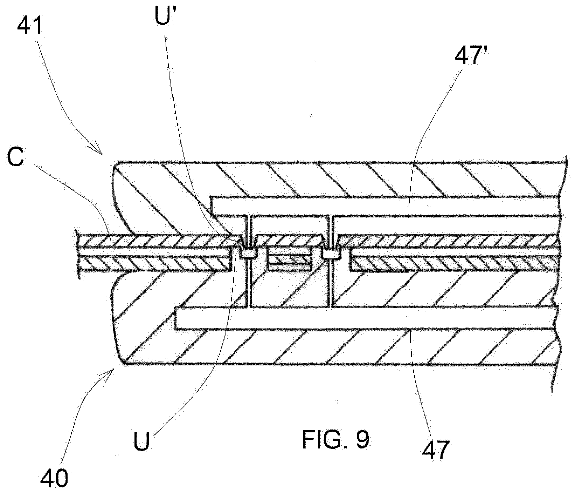

As shown in Fig. 9, advantageously the first longitudinal stretch 45 of the second jaw 41 also has needle-shaped nozzles U' which allow the micro-perforation of the colon part. The nozzles U' of the second jaw 41 couple with the nozzles U of the first jaw so as to pierce the tissue of the colon C and maintain air-tightness towards the outside, so that the washing liquid injected both by the nozzles U of the first jaw and by the nozzles U' of the second jaw remains inside the colon C and does not come out of the colon C.

As previously said, the device according to the invention ensures that the washing liquid has only the possibility of spreading inside the colon lumen and not in free peritoneum. This is ensured by the fact that externally the jaws 40, 41 are sealed, i.e., totally able to contain the washing liquid which spreads inside its internal duct.

The nozzles U, U' of the first and second jaws are connected to respective ducts 47, 47' obtained in the first jaw 40 and in the second jaw 41 of the second head. The ducts 47, 47' of the first jaw and of the second jaw are connected to a main duct 75 obtained in the shaft assembly G.

With reference to Fig. 5, the main duct 75 of the shaft assembly G is connected to a supply unit B, such as e.g. a pump supply unit or piston supply unit, adapted to supply a washing liquid that comes out of the nozzles U.

Each jaw of the forceps of the second head 4 has a rectangular-plate shape, having width W2 of about 3 - 6 mm, preferably 5 mm. The length of each jaw of the forceps 4 must be greater than the diameter of a colon-rectum stump, consequently the length of each jaw is about 30-35 mm. The thickness of each jaw of the forceps 4 is about 1-2 mm.

Each jaw of the stapler of the first head 3 has a rectangular-plate shape, having width W1 of about 5 - 9 mm, preferably 8 mm. The length of each jaw of the stapler 3 is equal to the length of the jaws of the forceps 4. The second jaw 31 of the stapler has a thickness substantially equal to the thickness of the jaws of the forceps 4. The first jaw 40 of the stapler, on the other hand, must have greater thickness than the second jaw, as in the first jaw the housing for the reload 33 has to be formed. In any case, the thickness of the first jaw of the stapler is about 4 - 6 mm.

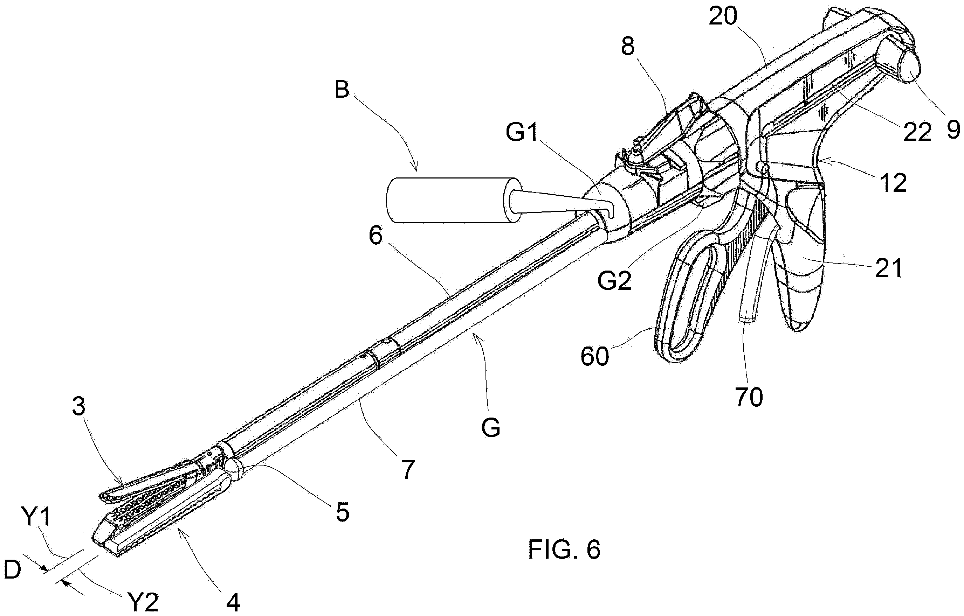

With reference to Fig. 6, the first head 3 has a longitudinal axis Y1 meant as the longitudinal median axis between the two closed jaws of the first head and the second head 4 has a longitudinal axis Y2 meant as the longitudinal median axis between the two second longitudinal stretches 46 of the two closed jaws of the second head. Between the axis of the first head 3 and the axis of the second head 4 there is a distance comprised between 4 - 7.5 mm, preferably 6.5 mm. In fact, with reference to Fig. 2, this distance D must correspond to the distance between the tightening plane A1 of the forceps of the second head 4 and the cut and suture plane A2 performed by the stapler of the first head 3. To keep this distance D, the first head 3 has width W1 of about 5 - 9 mm and the second head 4 has width W2 of about 3 - 6 mm.

The shaft assembly G comprises actuation rods (known in themselves and therefore not shown in the drawings) mounted in a sliding manner within the shaft assembly. The shaft assembly comprises:
- a first actuation rod that actuates the opening/closing of the jaws 30, 31 of the stapler;
- a second actuation rod that actuates the opening/closing of the jaws 40, 41 of the forceps.

Generally, the first jaw 30, 40 of the stapler and of the forceps is fixed, whereas the second jaw 31, 41 is movable. The actuation rods then actuate the second jaw 31, 41 of the stapler and of the forceps.

The shaft assembly G can have two separate external tubes 6, 7 connected to the two heads 3, 4 respectively, as shown in Fig. 5, or can have a single external tube. The main duct 75 for the washing fluid is in the external tube 7.

The handle 2 comprises a body 20 from which protrudes a grip 21 adapted to be gripped by the user.

A first trigger 60 is hinged to the handle body so as to be opposite the grip 21 to be operable by the surgeon. The first trigger 60 is connected to the first actuation rod, so as to allow opening/closing the stapler 3.

A second trigger 70 is hinged to the handle body so as to be opposite the grip, alongside the first trigger, to be operable by the surgeon. The second trigger 70 is connected to the second actuation rod, so as to allow the opening/closing of the forceps 4. Fig. 5 illustrates the forceps 4 open and Fig. 6 illustrates the forceps 4 closed.

Advantageously, the shaft assembly G comprises a shank G1 which is connected, by means of a ring G2, to the body 20 of the handle. The rotation of the ring G2 allows rotating the shaft assembly G around its axis, and therefore also the heads 3, 4, in accordance with the position of the surgical operation site. The supply unit B of the washing fluid, has a connector B1 which engages in the shank G1 of the shaft assembly, to connect to the main duct 75 formed inside the shaft assembly.

Advantageously the two heads 3, 4 are hinged to the shaft assembly G, by means of an articulated joint 5, so as to be able to vary the inclination of the two heads 3, 4 with respect to the axis of the shaft assembly G. For this purpose, an inclination adjustment rod (known in itself and therefore not shown) is arranged within the shaft assembly G to operate the articulated joint 5, so as to vary the inclination of the heads 3, 4 with respect to the axis of the shaft assembly.

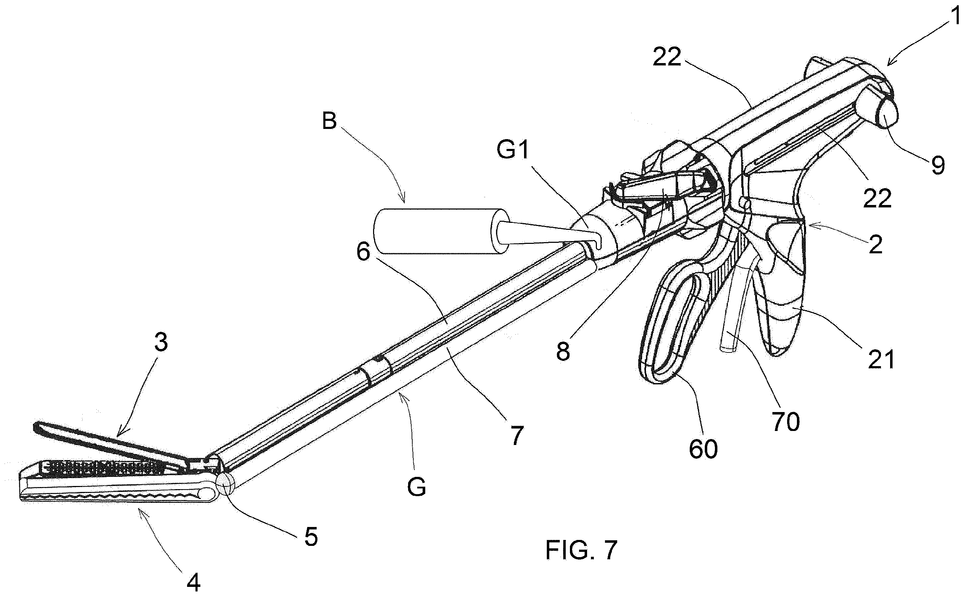

An adjustment lever 8, operable by the user, is connected to the inclination adjustment rod to operate the articulated joint 5 and vary the inclination of the heads 3, 4 in accordance with the position of the surgical operating site. In the illustrations, the lever 8 is arranged on the shank G1 of the shaft assembly; nevertheless, the lever 8 could also be arranged on the body 20 of the handle. Fig. 7 shows a configuration wherein the axis of the heads 3, 4 is inclined with respect to the axis of the shaft assembly G.

Although not shown in the figures, two articulated joints 5 can be provided which are connected to the first head 3 and to the second head 4 respectively, and two levers 8 connected to the two articulated rods. This way the inclination of the first head 3 and of the second head 4 can be independently adjusted.

A knob 9 is fitted sliding on the body 20. For example, the knob 9 slides in a longitudinal slot 22 formed in the body 20. The knob 9 is connected to an extension rod that slides longitudinally in the shaft assembly G. The extension rod is connected to the heads 3, 4. The knob 9 is operable by the surgeon, so as to adjust the length of the surgical instrument, and therefore the distance between the heads 3, 4 and the handle 2, in accordance with the position of the surgical operation site. Two knobs 9 can be provided connected to respective extension rods, connected to the first head 3 and to the second head 4 respectively, to independently adjust the position of the first head and of the second head.

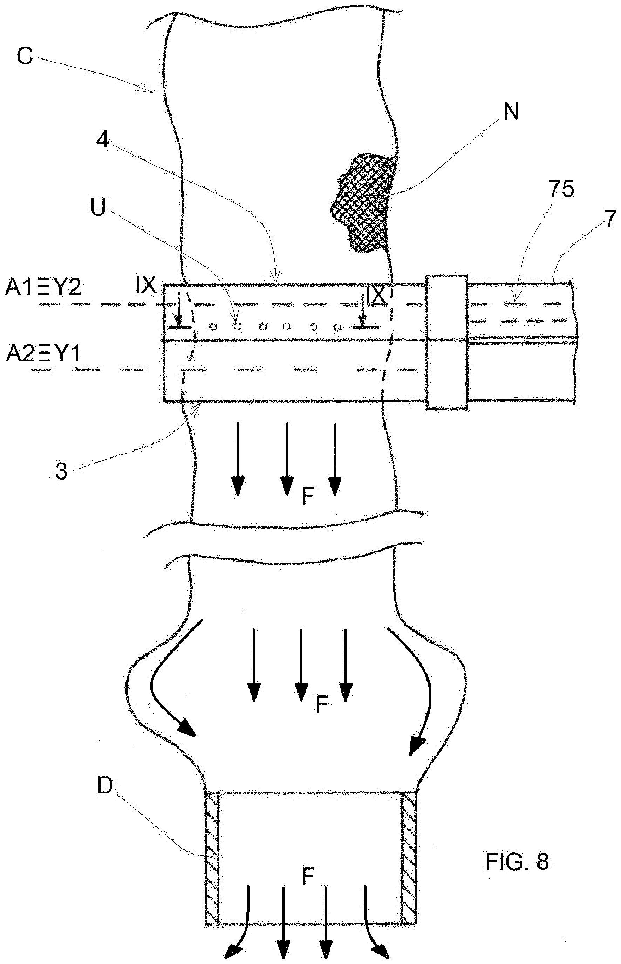

With reference to Fig. 8, the heads 3, 4 of the surgical instrument 1 are inserted by laparoscopy inside the patient's abdomen until they reach the part of the colon-rectum C where the neoplasia N is located.

Initially, the heads 3, 4 are open. The second head 4 is arranged under the neoplasia N. The first head 3 is under the second head 4.

The second head 4 is closed. As a result, the serrated surfaces 43 of the second head tighten the colon-rectum C stump along the median plane A1, without cutting the colon-rectum stump.

Then the nozzles U, U' of the second head are below the median plane A1.

The nozzles U, U' pierce the colon-rectum stump. At this point, the supply unit B is operated which supplies washing fluid under pressure which comes out of the nozzles U, U', and is injected into the colon-rectum stump. Since the median plane A1 above the nozzles U, U' is tightened by the serrated surfaces 43 of the jaws of the second head, the washing liquid flows downward, in the direction of the arrows F carrying out the efficient washing of the colon-rectum stump below the median plane A1.

To facilitate the exit of the washing liquid from the terminal section of the rectum, a retractor D can be fitted in the terminal section of the rectum.

Once the colon-rectum section has been washed below the median plane A1, the first head 3 is closed so as to cut and suture the colon-rectum along the lower plane A2 below the median plane A1, and therefore in a perfectly clean section of the colon-rectum.

## Claims

1. A surgical instrument (1) for colon-rectum operations comprising:
- a handle (2),
- a shaft assembly (G) that frontally protrudes from the handle,
- a first head (3) that frontally protrudes from the shaft assembly (G), said first head (3) comprising a mechanical stapler of 'cut-and-sew' type, adapted to cut and suture the colon-rectum;
- a second head (4) that frontally protrudes from the shaft assembly (G) disposed at the side of the first head (3), said second head (3) comprising forceps adapted to tighten and strangulate the colon-rectum without cutting it;
wherein said shaft assembly (G) and said first and second heads (3, 4) are dimensioned in such manner to be inserted into a cannula of a trocar for laparoscopy, **characterized by** the fact that said forceps of the second head (4) comprise a plurality of nozzles (U, U') connected to at least a duct (47, 47') formed in the second head (4) and communicating with a main duct (75) formed in said shaft assembly (G) connected to a supply unit (B) adapted to supply a washing liquid that comes out of the nozzles (U, U') to wash the colon-rectum stump from top to bottom, said nozzles (U, U') being configured so as to pierce the wall of the colon-rectum, but not cross the colon-rectum from side to side.

2. The surgical instrument (1) of claim 1, wherein said forceps of the second head (4) comprise: a first jaw (40) and a second jaw (41) hinged to the first jaw; the two jaws of the forceps have a first longitudinal stretch (45) near the first head (3) and a second longitudinal stretch (46) parallel to the first longitudinal stretch and further away from the first head, the second longitudinal stretches (46) of the two jaws have opposite and complementary serrated surfaces (43), adapted to tighten the colon-rectum stump, so as to strangulate without cutting it, said nozzles (U, U') being arranged in the first longitudinal stretch (45) of the first jaw (40) and/or of the second jaw (41),
said first longitudinal stretches (45) of the first jaw and of the second jaw (41) comprising an abutment surface adapted to interface with the nozzles (U, U') so as to ensure the nozzles (U, U') pierce the colon-rectum, but not cross the colon-rectum from side to side.

3. The surgical instrument (1) of claim 2, wherein the nozzles (U, U') are arranged in an intermediate position of the first stretch (45) of the first jaw and/or of the second jaw, the distal and proximal ends of the first stretch (45) of the first jaw and of the second jaw are therefore without nozzles, to prevent the washing liquid from being injected out of the colon-rectum.

4. The surgical instrument (1) of any one of the preceding claims, wherein said stapler of the first head (3) and said forceps of the second head (4) are disposed side-by-side and dimensioned in such manner that the longitudinal axes are spaced by a distance (D) comprised between 4 and 9 mm, preferably 6.5 mm.

5. The surgical instrument (1) of claim 4, wherein said stapler of the first head (3) has width (W1) comprised between 5 and 11 mm, preferably 8, and said forceps of the second head (4) have width (W2) comprised between 3 and 7 mm, preferably 5 mm.

6. The surgical instrument (1) of any one of the preceding claims, wherein said stapler of the first head (3) comprises a first jaw (30) and a second jaw (31) hinged to the first jaw; said first jaw housing a reload (33) comprising a cutting blade and suture clips, said forceps of the second head (4) comprise a first jaw (40) and a second jaw (41) hinged to the first jaw.

7. The surgical instrument (1) of claim 6, wherein said handle (2) comprises:
- a first trigger (60) connected to the stapler of the first head (3) by means of a first actuation rod in order to open/close the jaws (30, 31) of the stapler, and
- a second trigger (70) connected to the forceps of the second head (4) by means of a second actuation rod in order to open/close the jaws (40, 41) of the forceps.

8. The surgical instrument (1) of any one of the preceding claims, comprising at least one knob (9) connected to the heads (3, 4) by means of at least one extension rod in order to adjust the distance of the heads (3, 4) from the handle (2).

9. The surgical instrument (1) of any one of the preceding claims, also comprising a metal ring (G2) that connects the handle (2) to the shaft assembly (G) in order to make the shaft assembly (G) rotate around its axis.

## Patentansprüche

1. Chirurgisches Instrument (1) für Dickdarm-Enddarm-Operationen, umfassend:
- einen Handgriff (2);
- eine Schaftanordnung (G), die stirnseitig aus dem Handgriff hervorsteht,
- einen ersten Kopf (3), der stirnseitig aus der Schaftanordnung (G) hervorsteht, wobei der erste Kopf (3) ein mechanisches Klammergerät vom "Schneiden und Nähen"-Typ umfasst, das zum Zuschneiden und Vernähen des Dickdarms-Enddarms eingerichtet ist;
- einen zweiten Kopf (4), der stirnseitig an der Seite des ersten Kopfes (3) angeordnet aus der Schaftanordnung (G) hervorsteht, wobei der zweite Kopf (3) Zangen aufweist, die eingerichtet sind, den Dickdarm-Enddarm zusammenzuziehen und abzuschnüren, ohne ihn abzuschneiden;
wobei die Schaftanordnung (G) und der erste und der zweite Kopf (3, 4) in einer solchen Weise dimensioniert sind, dass sie in eine Kanüle eines Trokars für die Laparoskopie eingeführt werden können, **dadurch gekennzeichnet , dass** die Zangen des zweiten Kopfes (4) eine Mehrzahl von Düsen (U, U') umfassen, die mit mindestens einem Kanal (47, 47') verbunden sind, der in dem zweiten Kopf (4) ausgebildet ist, und mit einem Hauptkanal (75) in Verbindung stehen, der in der Schaftanordnung (G) ausgebildet ist, welcher mit einer Versorgungseinheit (B) verbunden ist, die zum Zuführen einer Waschflüssigkeit eingerichtet ist, die aus den Düsen (U, U') austritt, um den Dickdarm-Enddarm-Stumpf von oben nach unten zu waschen, wobei die Düsen (U, U') so konfiguriert sind, dass sie die Wand des Dickdarms-Enddarms durchstechen, jedoch nicht den Dickdarm-Enddarm von einer Seite zur anderen Seite durchqueren.

2. Chirurgisches Instrument (1) nach Anspruch 1, wobei die Zangen des zweiten Kopfes (4) umfassen: eine erste Klemmbacke (40) und eine zweite Klemmbacke (41), die an der ersten Klemmbacke angelenkt ist; wobei die beiden Klemmbacken der Zangen einen ersten Längsabschnitt (45) nahe von dem ersten Kopf (3) und einen zweiten Längsabschnitt (46) parallel zu dem ersten Längsabschnitt und weiter weg von dem ersten Kopf aufweisen, wobei die zweiten Längsabschnitte (46) der beiden Klemmbacken gegenüberliegende und komplementäre gezahnte Oberflächen (43) aufweisen, die so eingerichtet sind, dass sie den Dickdarm-Enddarm-Stumpf zusammenziehen, um ihn abzuschnüren, ohne ihn abzuschneiden, wobei die Düsen (U, U') in dem ersten Längsabschnitt (45) der ersten Klemmbacke (40) und / oder der zweiten Klemmbacke (41) angeordnet sind,
wobei die ersten Längsabschnitte (45) der ersten Klemmbacke und der zweiten Klemmbacke (41) eine Anlagefläche aufweisen, die eingerichtet ist, sich mit den Düsen (U, U') zu verbinden, um zu gewährleisten, dass die Düsen (U, U') den Dickdarm-Enddarm durchstechen, jedoch nicht den Dickdarm-Enddarm von einer Seite zur anderen Seite durchqueren.

3. Chirurgisches Instrument (1) nach Anspruch 2, wobei die Düsen (U, U') in einer mittleren Position von dem ersten Abschnitt (45) der ersten Klemmbacke und / oder der zweiten Klemmbacke angeordnet sind, wobei die distalen und proximalen Enden des ersten Abschnitts (45) von der ersten Klemmbacke und der zweiten Klemmbacke demzufolge ohne Düsen sind, um zu verhindern, dass die Waschflüssigkeit aus dem Dickdarm-Enddarm eingespritzt wird.

4. Chirurgisches Instrument (1) nach einem der vorhergehenden Ansprüche, wobei das Klammergerät des ersten Kopfes (3) und die Zangen des zweiten Kopfes (4) Seite an Seite angeordnet und in einer solchen Weise dimensioniert sind, dass die Längsachsen mit einem Abstand (D), der zwischen 4 und 9 mm liegt und vorzugsweise 6,5 mm beträgt, beabstandet sind.

5. Chirurgisches Instrument (1) nach Anspruch 4, wobei das Klammergerät des ersten Kopfes (3) eine Breite (W1) zwischen 5 und 11 mm, vorzugsweise 8, aufweist und die Zangen des zweiten Kopfes (4) eine Breite (W2) aufweisen, die zwischen 3 und 7 mm liegen und vorzugsweise 5 mm beträgt.

6. Chirurgisches Instrument (1) nach einem der vorhergehenden Ansprüche, wobei das Klammergerät des ersten Kopfes (3) eine erste Klemmbacke (30) und eine zweite Klemmbacke (31) aufweist, die an der ersten Klemmbacke angelenkt ist; wobei in der ersten Klemmbacke eine Nachladeeinrichtung (33) untergebracht ist, die eine Schneidklinge und Nahtklammern aufweist, wobei die Zangen des zweiten Kopfes (4) eine erste Klemmbacke (40) und eine zweite Klemmbacke (41) aufweist, die an der ersten Klemmbacke angelenkt ist.

7. Chirurgisches Instrument (1) nach Anspruch 6, wobei der Handgriff (2) umfasst:
- einen ersten Auslöser (60), der mit dem Klammergerät des ersten Kopfes (3) mittels einer ersten Betätigungsstange verbunden ist, um die Klemmbacken (30, 31) des Klammergerätes zu öffnen / zu schließen, und
- einen zweiten Auslöser (70), der mit dem Klammergerät des ersten Kopfes (4) mittels einer zweiten Betätigungsstange verbunden ist, um die Klemmbacken (40, 41) des Klammergerätes zu öffnen / zu schließen.

8. Chirurgisches Instrument (1) nach einem der vorhergehenden Ansprüche, umfassend mindestens einen Knopf (9), der mit den Köpfen (3, 4) mittels mindestens einer Verlängerungsstange verbunden ist, um den Abstand der Köpfe (3, 4) von dem Handgriff (2) einzustellen.

9. Chirurgisches Instrument (1) nach einem der vorhergehenden Ansprüche, das ebenfalls einen Metallring (G2) aufweist, der den Handgriff (2) mit der Schaftanordnung (G) verbindet, um die Schaftanordnung (G) um ihre Achse rotieren zu lassen.

## Revendications

1. Instrument chirurgical (1) pour des opérations du côlon-rectum comprenant :
- un manche (2),
- un ensemble arbre (G) qui dépasse frontalement du manche,
- une première tête (3) qui dépasse frontalement de l'ensemble arbre (G), ladite première tête (3) comprenant une agrafeuse mécanique de type « coupe et coud », adaptée pour couper et suturer le côlon-rectum ;
- une seconde tête (4) qui dépasse frontalement de l'ensemble arbre (G) disposé sur le côté de la première tête (3), ladite seconde tête (3) comprenant des forceps adaptés pour serrer et stranguler le côlon-rectum sans le couper ;
dans lequel ledit ensemble arbre (G) et lesdites première et seconde têtes (3, 4) sont dimensionnés de manière à être insérés dans une canule d'un trocart de laparoscopie, **caractérisé par le fait que** lesdits forceps de la seconde tête (4) comprennent une pluralité de buses (U, U') reliées à au moins un conduit (47, 47') formé dans la seconde tête (4) et communiquant avec un conduit principal (75) formé dans ledit ensemble arbre (G) relié à une unité de fourniture (B) adaptée pour fournir un liquide de lavage qui sort des buses (U, U') pour laver le moignon de côlon-rectum de haut en bas, lesdites buses (U, U') étant configurées de façon à percer la paroi du côlon-rectum, mais à ne pas traverser le côlon-rectum d'un côté à l'autre.

2. Instrument chirurgical (1) selon la revendication 1, dans lequel lesdits forceps de la seconde tête (4) comprennent : une première mâchoire (40) et une seconde mâchoire (41) articulée à la première mâchoire ; les deux mâchoires des forceps ont une première portion longitudinale (45) près de la première tête (3) et une seconde portion longitudinale (46) parallèle à la première portion longitudinale et davantage éloignée de la première tête, les secondes portions longitudinales (46) des deux mâchoires ont des surfaces crénelées opposées et complémentaires (43), adaptées pour serrer le moignon de côlon-rectum, de façon à le stranguler sans le couper, lesdites buses (U, U') étant agencées dans la première portion longitudinale (45) de la première mâchoire (40) et/ou de la seconde mâchoire (41),
lesdites premières portions longitudinales (45) de la première mâchoire et de la seconde mâchoire (41) comprenant une surface de butée adaptée pour servir d'interface avec les buses (U, U') de façon à garantir que les buses (U, U') percent le côlon-rectum, mais ne traversent pas le côlon-rectum d'un côté à l'autre.

3. Instrument chirurgical (1) selon la revendication 2, dans lequel les buses (U, U') sont agencées dans une position intermédiaire de la première portion (45) de la première mâchoire et/ou de la seconde mâchoire, les extrémités distale et proximale de la première portion (45) de la première mâchoire et de la seconde mâchoire sont par conséquent sans buses, pour empêcher le liquide de lavage d'être injecté hors du côlon-rectum.

4. Instrument chirurgical (1) selon l'une quelconque des revendications précédentes, dans lequel ladite agrafeuse de la première tête (3) et lesdits forceps de la seconde tête (4) sont disposés côte à côte et dimensionnés de manière à ce que les axes longitudinaux soient espacés d'une distance (D) comprise entre 4 et 9 mm, de préférence 6,5 mm.

5. Instrument chirurgical (1) selon la revendication 4, dans lequel ladite agrafeuse de la première tête (3) a une largeur (W1) comprise entre 5 et 11 mm, de préférence 8, et lesdits forceps de la seconde tête (4) ont une largeur (W2) comprise entre 3 et 7 mm, de préférence 5 mm.

6. Instrument chirurgical (1) selon l'une quelconque des revendications précédentes, dans lequel ladite agrafeuse de la première tête (3) comprend une première mâchoire (30) et une seconde mâchoire (31) articulée sur la première mâchoire; ladite première mâchoire logeant une recharge (33) comprenant une lame coupante et des agrafes, lesdits forceps de la seconde tête (4) comprennent une première mâchoire (40) et une seconde mâchoire (41) articulée à la première mâchoire.

7. Instrument chirurgical (1) selon la revendication 6, dans lequel ledit manche (2) comprend :
- une première détente (60) reliée à l'agrafeuse de la première tête (3) au moyen d'une première tige d'actionnement afin d'ouvrir/fermer les mâchoires (30, 31) de l'agrafeuse, et
- une seconde détente (70) reliée aux forceps de la seconde tête (4) au moyen d'une seconde tige d'actionnement afin d'ouvrir/fermer les mâchoires (40, 41) des forceps.

8. Instrument chirurgical (1) selon l'une quelconque des revendications précédentes, comprenant au moins un bouton (9) relié aux têtes (3, 4) au moyen d'au moins une tige d'extension afin de régler la distance entre les têtes (3, 4) et le manche (2).

9. Instrument chirurgical (1) selon l'une quelconque des revendications précédentes, comprenant également une bague en métal (G2) qui relie le manche (2) à l'ensemble arbre (G) afin de faire tourner l'ensemble arbre (G) autour de son axe.
